# FASCICULE DE BREVET EUROPEEN

(11) **EP 1 437 965 B1**
(45) Date de publication et mention de la délivrance du brevet: **04.04.2012**
(21) Numéro de dépôt: 02795323.1
(22) Date de dépôt: 16.10.2002
(51) Int. Cl.: A61B 8/08

(54) **PROCEDE POUR MESURER L'ETAT DE TENSION D'UN MATERIAU ET APPLICATIONS DE CE PROCEDE**
VERFAHREN ZUR MESSUNG VON SPANNUNGSZUSTÄNDEN EINES MATERIALS UND SEINE ANWENDUNG
METHOD FOR MEASURING TENSION STATE OF A MATERIAL AND USES THEREOF

(30) Priorité: 16.10.2001 FR 0113327
(43) Date de publication de la demande: 21.07.2004
(73) Titulaire: INSTITUT NATIONAL DE LA RECHERCHE AGRONOMIQUE (INRA), 75338 Paris Cédex 07 (FR); Ecole Nationale Veterinaire D'Alfort (ENVA), F-94704 Maisons-alfort Cedex (FR)
(72) Inventeur: POURCELOT, Philippe, F-37250 Veigne (FR); CREVIER-DENOIX, Nathalie, F-94340 Joinville-le-pont (FR); RAVARY, Bérangère, F-94700 Maisons-Alfort (FR)
(74) Mandataire: Catherine, Alain
(86) Numéro de dépôt international: PCT/FR2002/003544
(87) Numéro de publication internationale: WO 2003/032842

(56) Documents cités:
- WO-A-99/45348
- US-A- 5 170 366
- US-A- 5 678 565
- US-A- 6 007 489
- US-B1- 6 193 658
- US-B1- 6 213 934

## Description

La présente invention se rapporte au domaine de la mesure de l'état de tension d'un matériau biologique comme un tendon, un ligament ou un muscle.

### ART ANTERIEUR

La mesure de l'état de tension d'un matériau biologique présente un intérêt considérable dans de nombreux domaines industriels.

Il est primordial, pour de nombreux dispositifs mécaniques, de connaître l'état de tension de leurs différents éléments au cours des différentes phases de leur fonctionnement, notamment afin d'évaluer la capacité de résistance mécanique de ces dispositifs durant leur utilisation courante. De tels dispositifs incluent les véhicules terrestres ou aériens, pour lesquels certaines pièces en matériau composite sont susceptibles de subir de fortes contraintes mécaniques lorsqu'ils sont en mouvement.

La détermination de l'état de tension d'un matériau biologique, et plus spécifiquement d'un tendon, d'un ligament ou d'un muscle en réponse à des sollicitations mécaniques exercées de façon statique, en contraction isométrique par exemple, ou de façon dynamique, lors de la locomotion normale ou pathologique ou encore au cours de différents mouvements ou gestes sportifs, présente aussi un grand intérêt technique.

Chez l'homme comme chez l'animal, la mise au point de chaussures, ou de fers adaptés aux divers types de sollicitations mécaniques spécifiques au type d'activité, par exemple la course, le saut, pourrait être optimisée si l'industriel avait à sa disposition une mesure simple et reproductible pour déterminer l'état de tension d'un tendon, d'un ligament ou d'un muscle aux différentes phases de mouvement du sujet dans l'activité considérée. L'accessibilité d'une telle mesure permettrait, par exemple, la conception de semelles, de fers ou de palmes ayant des caractéristiques mécaniques optimisées pour la pratique d'un sport en particulier.

De même, une telle mesure permettrait d'étudier ou de sélectionner des dispositifs correcteurs de la marche, de la course ou de tout autre geste statique ou dynamique, ceci pour des tissus tendineux, ligamentaires ou musculaires lésés ou en phase de rééducation comme des semelles correctrices chez l'Homme ou des ferrures correctrices chez les Equidés.

Une telle mesure permettrait également d'aider à la conception de nouveaux matériels sportifs ou médicaux, comme des chaussures ou des fers, des palmes, des fixations de skis, des appareils de musculation, notamment.

Des études *in vitro* ont permis d'évaluer les charges et les déformations qui s'exercent sur les tendons de la main du cheval au cours des différentes phases de la foulée. On peut citer notamment les travaux de Jansen et *al*. et de Riemersma et *al*. (Jansen MO et al., In vivo tendon forces in the forelimb of ponies at the walk validated by ground reaction forces measurements, Acta Anatomica, 1993, 146:162-167 ; Riemersma DJ et al, Influence of shoeing on ground reaction forces and tendon strains in the forelimb in ponies, Equine Vet. J., 1996, 28(2):126-132; Riemersma DJ et al., Tendon strain in the forelimbs as a function of gait and ground characteristics and in vitro limb loading in ponies, Equine Vet. J., 1996, 28(2):133-138 ; Riemersma DJ et al., Kinetics and kinematics of the equine hindleg : II, in vivo tendon strain and joint kinematics, Am. J. Vet. Res., 1988, 49:1353-1359) qui utilisent des capteurs intra-tendineux à jauge de déformation ou encore les travaux de Stephens et *al*. (Stephens PR et al., Application of a Hall-effect transducer for measurement of tendon strain in Horses, Am. J. Vet. Res., 1989, 50:1089-1095) utilisant des capteurs à effet Hall.

Il a également été proposé de mesurer la force exercée sur un tendon à l'aide de fibres optiques (Erdemir et al., In vitro evaluation of a fiberoptic transducer for tendon force measurements, Congrès de la Société Internationale de Biomécanique, Zurich, Juillet 2001).

La nature invasive ou intrusive des capteurs utilisés dans l'état de la technique engendre non seulement des problèmes de nature éthique, mais aussi de nombreuses difficultés méthodologiques.

Ainsi, l'implantation de tels capteurs, qui nécessite une anesthésie générale, affecte les propriétés mécaniques locales des tendons en quelques jours, ce qui fausse la mesure de manière importante. Par ailleurs, la souffrance de l'animal est telle que seules des mesures aux allures lentes (pas et trot) peuvent en général être réalisées. Enfin, pour des raisons liées à la calibration des mesures, l'animal doit être euthanasié au terme de l'expérimentation. Il existe donc un besoin dans l'état de la technique pour la mise au point d'un procédé qui puisse constituer un « capteur *in vivo* » et qui permette une mesure directe et non invasive de l'état de tension d'un tendon, d'un ligament ou d'un muscle.

Il est désormais fourni selon l'invention un procédé simple et reproductible de détermination de l'état de tension d'un matériau biologique basé sur le calcul de la valeur d'au moins un paramètre extrait à partir du signal ultrasonore reçu après propagation d'une onde ultrasonore dans ledit matériau.

Les ultrasons sont utilisés depuis une dizaine d'années pour caractériser les matériaux biologiques ou non biologiques. Les systèmes d'échographie constituent une illustration de l'utilisation des ultrasons en imagerie médicale. Par exemple, la demande de brevet US N°6,193,658 délivrée le 27 Février 2001 décrit un dispositif à ultrasons utilisable pour mesurer l'étendue d'une lésion dans les tissus mous et les os, y compris les tendons, les ligaments et les muscles. Un tel dispositif est adapté à la détection et à la mesure de lésions caverneuses, après le remplissage de la lésion caverneuse avec un milieu fluide avant obstruction de la lésion à l'aide d'un film sur lequel sera appliqué le dispositif de mesure d'ultrasons.

Le brevet US N°5,685,307 délivré le 11 Novembre 1997 décrit un procédé de mesure par ultrasons de la masse graisseuse sur la carcasse d'un animal.

L'effet Doppler a également été mis à profit pour suivre les battements du coeur par analyse des échos d'ultrasons, comme cela est décrit notamment dans la demande de brevet européen N°EP 1 079 240.

La mesure de la: vitesse d'ultrasons a également été utilisée pour détecter des micro-fractures ou des déformations au niveau des os, pour estimer la densité osseuse, pour évaluer le processus de guérison osseuse ou pour mesurer l'épaisseur du cortex osseux, comme cela est décrit dans la demande PCT N°WO 99/45 348.

On a aussi proposé de mesurer le temps de parcours des ultrasons pour évaluer la déformation d'une structure rigide en fonction des contraintes mécaniques qui sont subies par cette structure rigide. Ainsi, le brevet américain n° US 5,170,366 décrit un procédé de mesure utilisant deux transducteurs ultrasonores indépendants, la mesure du temps de parcours des ultrasons permettant d'évaluer la distance entre les deux transducteurs et ainsi déterminer la déformation du matériau rigide sous l'effet d'une contrainte mécanique. Selon le procédé décrit dans ce brevet, les deux transducteurs ultrasonores sont chacun fixés en un point donné sur le matériau rigide testé.

### DESCRIPTION DÉTAILLÉE DE L'INVENTION

Il est désormais montré selon l'invention que l'état de tension d'un matériau peut être déterminé par la mesure d'au moins un paramètre extrait du signal ultrasonore reçu après propagation d'une onde ultrasonore dans ledit matériau.

L'invention est un procédé tel que défini dans la revendication 1.

Par « état de tension » d'un matériau, on entend selon l'invention, l'état physique qui caractérise ce matériau, qui résulte de l'ensemble des contraintes, notamment mécaniques (exemples : force, déformation), subies par ledit matériau, à un instant donné.

Selon l'invention; la variation d'un paramètre extrait du signal ultrasonore est « proportionnelle » à la variation de l'état de tension du matériau testé lorsque la valeur dudit paramètre augmente avec l'accroissement de l'état de tension du matériau et lorsque la valeur dudit paramètre décroît avec la diminution de l'état de tension du matériau.

Selon l'invention, la variation d'un paramètre extrait du signal ultrasonore est « inversement proportionnelle » à la variation de l'état de tension du matériau testé lorsque la valeur dudit paramètre diminue avec l'accroissement de l'état de tension du matériau et lorsque la valeur dudit paramètre s'accroît avec la diminution de l'état de tension du matériau.

Cela ne signifie pas nécessairement que la valeur du paramètre extrait du signal ultrasonore soit un multiple simple de la valeur de l'état de tension du matériau. La relation entre la valeur du paramètre extrait du signal ultrasonore et la valeur de l'état de tension du matériau peut aussi consister en une fonction mathématique complexe.

La fonction mathématique reliant la variation du paramètre extrait du signal ultrasonore et la variation de l'état de tension du matériau testé peut être conventionnellement déterminée par l'homme du métier.

De plus, la connaissance de la fonction mathématique reliant la variation de la valeur du paramètre extrait du signal ultrasonore à la variation de l'état de tension du matériau testé n'est pas nécessairement requise. Pour chacun des paramètres considérés, l'homme du métier peut établir une courbe étalon avec un premier axe correspondant aux valeurs du paramètre extrait du signal ultrasonore et un second axe correspondant aux valeurs d'état de tension du matériau testé. La courbe étalon est construite en appliquant au matériau testé une série de contraintes mécaniques de valeurs connues, par exemple une série de charges de valeurs connues, et en mesurant, pour chaque point de cette contrainte mécanique la valeur du paramètre extrait du signal sonore. Puis, pour l'évaluation de l'état de tension du matériau testé, à un instant donné, on mesure la valeur du paramètre extrait du signal ultrasonore et l'on reporte cette mesure sur la courbe étalon préétablie afin de déterminer la valeur de la contrainte mécanique (par exemple la charge) du matériau testé à cet instant donné.

Alternativement, la détermination quantitative de la valeur de l'état de tension du matériau testé, à un instant donné, n'est pas toujours requise. Dans de nombreuses situations, ce qui est important est la détermination de la variation de l'état de tension dudit matériau entre deux instants de mesure ou entre deux conditions de mesure distinctes et donc simplement la comparaison de la valeur de tension obtenue, entre ces deux instants, par mesure à chacun des deux instants ou pour chacune des deux conditions de mesure de la valeur du paramètre extrait du signal ultrasonore.

Le procédé ci-dessus est caractérisé en ce que le ou les paramètre(s) calculé(s) à partir du signal ultrasonore reçu après propagation de l'onde ultrasonore dans le matériau est (sont) choisi(s) parmi les paramètres suivants :
(i) le temps de parcours de l'onde ultrasonore sur une distance prédéterminée dans ledit matériau;
(ii) l'amplitude de l'onde ultrasonore au point de réception du signal ultrasonore ;
(iii) l'atténuation de l'onde ultrasonore au point de réception du signal ultrasonore ;
(iv) la fréquence moyenne de l'onde ultrasonore au point de réception du signal ultrasonore ;
(v) la fréquence maximale de l'onde ultrasonore au point de réception du signal ultrasonore.

Il a en particulier été montré selon l'invention que le temps de parcours, l'amptitude et l'atténuation des ultrasons parcourant un tissu du type tendon, ligament ou muscle, varient en fonction de l'état de tension de ce tissu.

Plus spécifiquement, il a été montré selon l'invention que l'amplitude d'une onde ultrasonore dans un tissu tendineux, ligamentaire ou musculaire augmente en fonction de la charge de tension exercée sur le tissu considéré. Il a aussi été montré que le temps de parcours et l'atténuation de l'onde ultrasonore décroissent en fonction de cette même charge. Cette dernière caractéristique a été montrée notamment pour l'homme et le cheval.

Le demandeur a aussi observé que la fréquence moyenne et la fréquence maximale de l'onde ultrasonore varient en fonction de l'état de tension du matériau, notamment du tendon, du ligament ou du muscle.

Sans vouloir être lié par une quelconque théorie, le demandeur pense que les variations concernant le temps de parcours, l'amplitude, l'atténuation, la fréquence moyenne et la fréquence maximale d'une onde ultrasonore peuvent être observées aussi sur des matériaux non biologiques, et plus spécifiquement sur des matériaux non biologiques dont la structure physique se rapproche de celle d'un tendon, d'un ligament ou d'un muscle, à savoir sur les matériaux non biologiques possédant une structure constituée de fibres orientées selon un axe principal. Cela s'applique en particulier aux matériaux non biologiques constitués de fibres incluses dans une matrice, comme dans les matériaux composites,

notamment les matériaux composites constitués de fibres de carbone, de fibres de verre ou de fibres de Kevlar®. L'invention ne concerne pas l'utilisation des matériaux non biologiques mentionnés.

Tirant parti de ces observations, le demandeur a mis au point un procédé pour déterminer l'état de tension d'un tendon, d'un ligament ou d'un muscle par mesure, ou par la mesure et le calcul, d'au moins un paramètre.

De préférence, le ou les paramètres extrait(s) du signal ultrasonore est (sont) choisi(s) parmi (i) le temps de parcours de l'onde ultrasonore sur une distance prédéterminée dudit tissu, (ii) l'amplitude de l'onde ultrasonore au point de réception du signal ultrasonore, (iii) l'atténuation, (iv) la fréquence moyenne et (v) la fréquence maximale du signal, ultrasonore au point de réception des ultrasons.

L'invention concerne aussi un procédé pour déterminer l'état de tension d'un tendon, d'un ligament ou d'un muscle à un instant donné comprenant une étape au cours de laquelle on calcule le temps de parcours ou l'amplitude ou l'atténuation d'une onde ultrasonore dans le tissu dudit tendon, dudit ligament ou dudit muscle, le temps de parcours, l'amplitude et l'atténuation étant fonction de la charge de tension dudit tendon, dudit ligament ou dudit muscle à cet instant donné.

Avantageusement, le procédé selon l'invention peut être réalisé en calculant la valeur d'une pluralité de paramètres extraits du signal ultrasonore, de préférence une pluralité de paramètres parmi ceux listés précédemment.

Préférentiellement, le procédé selon l'invention est caractérisé en ce que l'on calcule les valeurs de paramètre pour au moins l'une des combinaisons de paramètres suivantes :
(i) le temps de parcours et l'amplitude de l'onde ultrasonore ;
(ii) le temps de parcours et l'atténuation de l'onde ultrasonore ;
(iii) l'amplitude et l'atténuation de l'onde ultrasonore ;
(iv) le temps de parcours, l'amplitude et l'atténuation de l'onde ultrasonore.

Contrairement aux procédés de l'état de la technique, le procédé ci-dessus permet de déterminer l'état de tension d'un tendon, d'un ligament ou d'un muscle sans étape invasive d'implantation de matériels de mesure tels que des jauges de déformation, des capteurs à effet Hall ou encore des fibres optiques.

De même, pour les matériaux non biologiques, le procédé de l'invention permet de réaliser des mesures de l'état de tension du matériau, par exemple une pièce d'avion, sans altérer sa structure. Après la mesure, le matériau, par exemple la pièce d'avion, peut être réutilisée normalement dans son état premier.

Selon le procédé, on applique un transducteur ultrasonore émetteur au contact du matériau à traiter, puis les signaux provenant de cet émetteur et qui ont' parcouru ledit matériau sur une longueur ou distance prédéterminée, sont reçus au moyen d'un ou de plusieurs transducteurs ultrasonores récepteurs.

Lorsque le procédé est réalisé sur un matériau biologique du type tendon, ligament ou muscle, on applique un transducteur ultrasonore émetteur au contact de la peau, en regard du tendon, du ligament ou du muscle pour lequel la mesure de l'état de tension est recherchée, puis les signaux provenant de cet émetteur sont reçus au moyen d'un ou de plusieurs transducteurs ultrasonores récepteurs.

Il est montré selon l'invention que la mesure des variations de temps de parcours, d'amplitude ou d'atténuation des ultrasons en fonction de la charge de tension du tendon, du ligament ou du muscle n'est pas altérée selon que les points d'émission et de réception des ultrasons sont appliqués directement sur le tendon, le ligament ou le muscle, ou bien simplement mis en contact avec la peau sus-jacente.

Le passage des ultrasons à travers la peau et les tissus conjonctifs du tendon, du ligament ou du muscle, n'affecte pas la mesure des variations de temps de parcours, d'amplitude ou d'atténuation des ultrasons.

En conséquence, le procédé selon l'invention ne nécessite pas de système complexe de correction de mesure tenant compte du trajet du faisceau d'ultrasons à travers la peau ou les tissus conjonctifs environnant le tissu dont la charge de tension est mesurée.

L'invention est relative à un procédé pour déterminer l'état de tension d'un matériau à un instant donné, caractérisé en ce qu'il comprend les étapes suivantes :
a) appliquer une source d'ultrasons au contact du matériau à tester et enregistrer les signaux provenant de cette source d'ultrasons après propagation dans ledit matériau à l'aide d'au moins un récepteur situé à une distance fixe prédéterminée de ladite source, la ligne reliant la source et le ou les récepteurs étant parallèle à l'axe longitudinal du matériau à tester.
b) déterminer l'état de tension du matériau en calculant la valeur du ou des paramètre(s) extrait(s) du signal ultrasonore à un instant donné.

Selon une caractéristique avantageuse du procédé, la source d'ultrasons et le ou les récepteurs d'ultrasons sont placés au contact du matériau à tester et alignés selon l'axe principal dudit matériau, en général l'axe longitudinal dudit matériau.

L'axe longitudinal du matériau à tester est l'axe principal d'orientation des fibres qui le constituent.

L'invention concerne aussi un procédé pour déterminer l'état de tension d'un tendon, d'un ligament ou d'un muscle, ledit procédé comprenant les étapes suivantes :
a) appliquer une source d'ultrasons au contact de la peau en regard d'un tendon, d'un ligament ou d'un muscle et recevoir les signaux provenant de cette source d'ultrasons après propagation dans ledit matériau à l'aide d'au moins un récepteur situé à une distance fixe, prédéterminée de ladite source, la source et le ou les récepteurs étant alignés selon l'axe principal du tendon, du ligament ou du muscle investigué,
b) déterminer l'état de tension du tendon, du ligament ou du muscle en calculant la valeur du ou des paramètres extrait(s) du signal ultrasonore à un instant donné.

De préférence, la source d'ultrasons et le ou les récepteurs permettant de recevoir les signaux provenant de la source ultrasonore consistent en des transducteurs ultrasonores conventionnels, respectivement un transducteur émetteur et un ou plusieurs transducteurs récepteurs bien connus dans l'état de la technique.

Selon une caractéristique avantageuse du procédé, la source d'ultrasons et le ou les récepteurs d'ultrasons sont placés au contact du matériau à tester et alignés selon l'axe principal dudit matériau.

Selon une caractéristique avantageuse du procédé ci-dessus, la source d'ultrasons et le ou les récepteurs d'ultrasons sont placés sur la peau selon l'axe principal du tissu investigué, le ou les récepteurs étant situés à une distance fixe et prédéterminée de la source d'émission d'ultrasons. Selon cette caractéristique avantageuse, la distance à parcourir par les ultrasons est constante au cours du temps lorsqu'une succession de mesures est effectuée, le seul paramètre variable étant alors l'état de tension du tissu investigué.

Selon une autre caractéristique avantageuse du procédé ci-dessus, l'axe sur lesquels sont placés la source d'émission d'ultrasons et le ou les récepteurs est l'axe principal du tissu investigué, c'est-à-dire l'axe selon lequel sont alignées les fibres tendineuses, ligamentaires ou musculaires. Selon cette caractéristique, on évite les artéfacts de mesures qui auraient pu résulter d'un trajet des ultrasons imposé par un placement des transducteurs perpendiculairement à l'axe des fibres tendineuses, ligamentaires ou musculaires, artéfacts liés aux variations de diamètre du tissu considéré (tendon, ligament ou muscle) selon la charge de tension appliquée audit tissu, notamment lors des contractions musculaires ou de l'allongement des tendons ou des ligaments.

Comme cela est illustré dans les exemples, des corrélations ont été établies entre la charge dé tension appliquée au tendon et le temps de parcours, l'amplitude et l'atténuation de l'onde ultrasonore dans le tissu de ce dernier. Au fur et à mesure que la charge de tension appliquée au tendon s'élève, l'amplitude augmente tandis que le temps de parcours et l'atténuation du signal ultrasonore diminuent.

Selon un mode de réalisation préféré du procédé, les signaux provenant de la source d'ultrasons sont reçus par une pluralité de récepteurs situés à une distance prédéterminée par rapport à la source.

Selon ce mode de réalisation, la source d'ultrasons et la pluralité de récepteurs sont alignées selon l'axe des fibres tendineuses, ligamentaires ou musculaires.

Avantageusement, la source d'ultrasons et le ou les récepteurs sont inclus dans un même boîtier, ce qui permet de maintenir durablement constante la distance qui sépare l'émetteur du ou des récepteurs comme les distances qui séparent les récepteurs entre eux.

Selon encore un autre mode de réalisation conforme à l'invention, ledit boîtier comprend une pluralité d'émetteurs d'ultrasons, les émetteurs étant disposés, par exemple dans le boîtier ci-dessus, selon un axe perpendiculaire à l'axe formé par l'alignement de l'émetteur et du récepteur ou la pluralité de récepteurs tel que décrit précédemment. Dans ce mode de réalisation particulier, chaque émetteur est aligné avec un récepteur ou une pluralité de récepteurs, les axes formés par l'alignement d'un émetteur donné et du ou des récepteur(s) correspondant(s) étant parallèles entre eux et perpendiculaires à l'axe formé par l'alignement des émetteurs. Selon ce mode de réalisation, les émetteurs et les récepteurs sont disposés selon une matrice, comme cela est décrit par exemple dans la demande internationale PCT publiée le 10 Septembre 1999 sous le numéro WO 99/45348.

Grâce à un tel dispositif, il est possible de sélectionner, pendant la mesure, l'émetteur et les récepteurs alignés avec ce dernier qui permettent la réception d'un signal optimal, ce qui permet de pallier d'éventuels dysfonctionnements provoqués par le déplacement du dispositif sur la surface du matériau à tester au cours de la mesure, par exemple lors de la mesure de l'état de tension d'un tendon d'un homme ou d'un animal pendant la course.

De préférence, le dispositif comprenant un émetteur et un ou une pluralité de récepteurs est relié à un boîtier électronique de commande et de traitement du signal ultrasonore. Ce boîtier électronique peut être lui-même relié à un ordinateur numérique dont la mémoire a préalablement était chargée d'un programme informatique capable de gérer l'émission des ultrasons et le stockage des signaux recueillis.

Le temps de parcours des ultrasons le long de l'axe principal du matériau à tester, notamment le long de l'axe principal du tendon, du ligament ou du muscle, peut être représenté comme le temps de parcours de l'onde ultrasonore entre l'émetteur et l'un au moins des récepteurs.

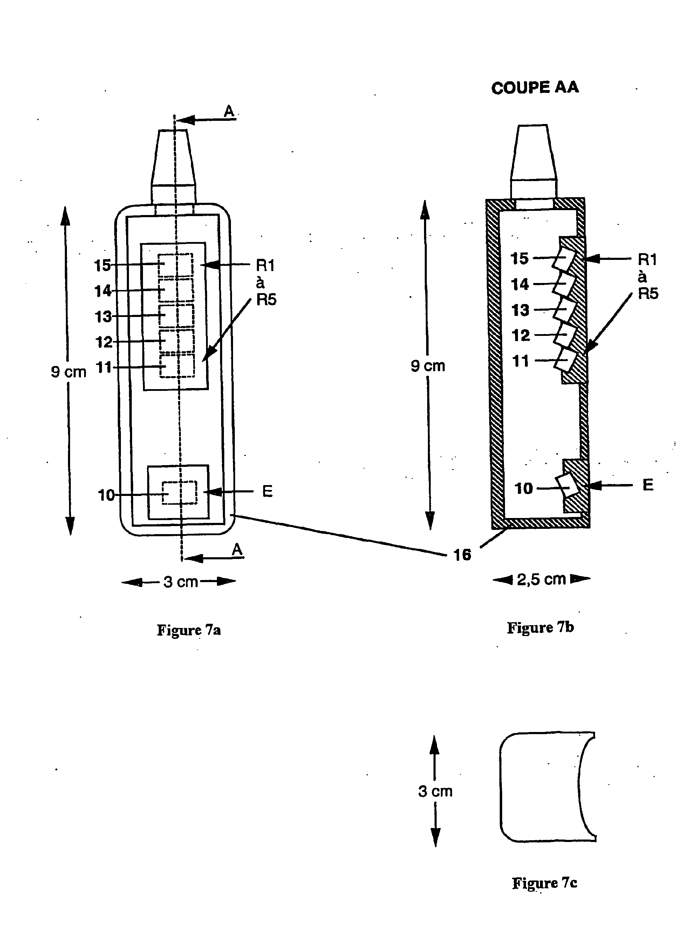

Selon un autre aspect, le temps de parcours de l'onde ultrasonore peut être représenté comme le temps de parcours du signal entre au moins deux récepteurs choisis parmi la pluralité de récepteurs. Selon cet aspect particulier, la précision de mesure est d'autant plus grande que le nombre de récepteurs entre lesquels le temps de parcours des ultrasons est mesuré est grand. Ainsi lorsque le dispositif de mesure comprend cinq récepteurs R₁ à R₅, comme cela est illustré dans les figures 7A et 7B, le temps de parcours de l'onde ultrasonore entré les récepteurs R₁ et R₅ peut être mesuré, puis exprimé en fonction de la distance séparant ces capteurs.

Dans ce mode de réalisation, on mesure le temps de parcours des ultrasons successivement entre le récepteur R₁ et les autres récepteurs c'est-à-dire successivement entre R₁ et R₂, R₁ et R₃, R₁ et R₄, R₁ et R₅.

Si l'on exprime ces mesures sous la forme d'une courbe dont l'abscisse est le temps de parcours mesuré entre R₁ et chacun des quatre autres récepteurs et l'ordonnée la distance entre R₁ et chacun des quatre autres récepteurs, alors la pente de la courbe ainsi obtenue est d'autant plus forte que la célérité des ultrasons est élevée.

Afin de mesurer la variation de temps de parcours des ultrasons en fonction de l'état de tension du tissu investigué, une autre méthode que celle décrite ci-dessus peut être employée. Constatant que le tracé des signaux acquis pour des charges croissantes ou décroissantes présente une évolution progressive à la fois dans leur phase et dans leur amplitude, cette méthode consiste à calculer le décalage temporel (retard ou avance) entre deux signaux enregistrés successivement en déterminant le maximum de la fonction d'intercorrélation de ces deux signaux sur une fenêtre donnée de temps.

La fenêtre de temps sur laquelle s'effectue l'intercorrélation peut varier de 1 à 50 microsecondes, préférentiellement 2 à 10 microsecondes pour une émission ultrasonore à 1 MHz.

Pour déterminer le maximum de la fonction d'intercorrélation de ces deux signaux sur une fenêtre donnée de temps, l'homme du métier peut avantageusement se référer à l'ouvrage de M. KUNT (1981, « Traitement numérique des signaux » DUNOD Ed., pp.16-17 et 57).

Pour s'affranchir de la résolution temporelle liée à la fréquence d'échantillonnage des signaux ultrasonores, on procède à l'interpolation du maximum de la fonction d'intercorrélation, par exemple par Fast Fourier Transform (ouvrage de M. Kunt, 1981, « Traitement numérique des signaux » DUNOD Ed., pp. 173-174) ou par interpolation parabolique (ouvrage de F. SCHEID, 1986, « Analyse numérique » Série Schaum, Mc Gray Hill Ed., pp 82-87 et 90-99) à partir des 3 points définissant le maximum.

Comme le temps de parcours, l'amplitude et l'atténuation de l'onde ultrasonore sont fonction de la charge exercée sur le tendon, le ligament ou le muscle. L'onde ultrasonore reçue par le transducteur ou les transducteurs ultrasonores est un signal complexe constitué d'une sommation d'ondes d'amplitudes et de fréquences distinctes. Il est montré selon l'invention que, pour une charge appliquée au matériau à tester, par exemple un tendon, le signal ultrasonore reçu par le ou les transducteurs peut être caractérisé notamment par une valeur d'amplitude, d'atténuation et de fréquence.

L'amplitude du signal ultrasonore peut être représentée comme la valeur maximale du module de la transformée de Fourier de ce signal, qui peut être calculée par l'homme du métier, notamment conformément à l'enseignement de l'ouvrage de M. KUNT (1981, « Traitement numérique des signaux » DUNOD Ed., pp. 133 et 165-170).

L'atténuation du signal ultrasonore peut être représentée comme la pente de la fonction d'atténuation (en dB/MHz) obtenue en rapportant les modules des spectres en fréquence à des modules pris comme référence. Les modules de référence pourront, par exemple, être ceux calculés pour la charge appliquée la plus élevée.

La fréquence du signal ultrasonore peut être, notamment, caractérisée par deux paramètres. La fréquence maximale peut être représentée comme la fréquence correspondant au maximum d'amplitude du module du spectre fréquentiel et la fréquence moyenne comme le barycentre du spectre fréquentiel.

L'amplitude de l'onde ultrasonore est d'autant plus élevée que la charge exercée sur le tissu investigué est grande. Inversement, l'atténuation de l'onde ultrasonore est d'autant plus faible que la charge exercée sur le tissu investigué est grande.

Pour le calcul du spectre fréquentiel et du spectre d'amplitude, l'homme du métier peut avantageusement se référer à l'ouvrage de M. KUNT (1981, « Traitement numérique des signaux », DUNOD Ed., pp.12-13).

Selon encore un autre aspect du procédé de l'invention, le demandeur a montré que l'angle entre l'émetteur et le ou les récepteurs avait une incidence significative, notamment sur l'amplitude de l'onde ultrasonore et a en conséquence une influence significative sur la précision des mesures.

Bien que l'angle entre l'émetteur d'ultrasons, d'une part, et le ou les récepteurs d'ultrasons, d'autre part, puisse varier de 0° à 180° et que l'angle optimal dépende notamment du matériau ou du tissu biologique investigué, de son impédance acoustique, de son épaisseur ou encore du nombre de couches à traverser pour l'atteindre, il est montré selon l'invention que, pour effectuer des mesures sur le tendon superficiel du doigt du cheval, les conditions opératoires optimales étaient atteintes lorsque cet angle était compris entre 20° et 160°.

De manière tout à fait préférée, l'angle formé entre l'émetteur d'ultrasons et le ou les récepteurs est compris entre 60° et 100° et encore plus préférentiellement est d'environ 80°.

Avantageusement, la fréquence des ultrasons émise par la source est comprise entre 15 KHz et 10 GHz, de préférence entre 20 KHz et 100 MHz, et de manière tout à fait préférée entre 20 KHz et 50 MHz.

De préférence, le procédé selon l'invention est caractérisé en ce que le ou les paramètre(s) extraits du signal ultrasonore reçu est (sont) calculé(s) entre au moins deux récepteurs.

En répétant le procédé ci-dessus pour une pluralité d'instants donnés, l'état de tension d'un matériau à tester, notamment d'un tendon, d'un ligament ou d'un muscle, peut être mesuré en continu sur le matériau dans les conditions de son utilisation ou de son fonctionnement, par exemple sur l'homme ou l'animal en mouvement ou non, afin de suivre en temps réel les charges de tension imposées au matériau ou au tissu biologique testé.

L'invention concerne aussi un procédé de mesure en continu de l'état de tension d'un matériau, caractérisé en ce qu'on met en oeuvre le procédé de détermination de l'état de tension d'un matériau tel que défini précédemment, pour une pluralité d'instants donnés, échelonnés dans le temps.

Lorsque le matériau à tester est un tendon, un ligament ou un muscle, le procédé ci-dessus peut être mis en oeuvre dans diverses situations de mouvement de l'Homme ou l'animal, notamment la locomotion (pas, trot, galop, course, saut, nage,), dans toutes les phases d'allures (amortissement, propulsion, ramener, embrassée, appel) ou dans n'importe quelle situation statique ou de mouvement dynamique.

De préférence, la fréquence de mesure de l'état de tension du tissu étudié est supérieure ou égale à 2,5 fois celle de la fréquence à laquelle s'effectue l'évolution de l'état de tension.

A titre illustratif, le cycle complet d'un membre, c'est-à-dire la foulée d'un cheval trottant au petit trot à une vitesse de 3 à 4 mètres par seconde, est réalisé, en moyenne, en 0.68 seconde, la phase d'appui durant environ 0.34 seconde.

Si l'on estime qu'au cours de cette phase d'appui la fréquence maximale à laquelle évolue la tension du tendon que l'on souhaite investiguer est de 40 Hz, il faudra effectuer 100 mesures par seconde pour déterminer l'état de tension de ce tendon durant la phase d'appui.

Le procédé ci-dessus peut être appliqué à la détermination de l'état de tension d'un tendon, d'un muscle ou d'un ligament, chez l'Homme ou l'animal à un instant donné, ou au suivi des variations de l'état de tension du tissu biologique considéré au cours du temps, par exemple pendant une locomotion (marche ou course) normale ou pathologique, ou encore pendant un geste sportif.

En particulier, le procédé selon l'invention peut être réalisé sur des tissus lésés, par exemple dans le cas d'inflammation ou autres lésions des tendons, ou encore après appareillage d'un membre avec une prothèse.

Chez l'Homme, le procédé ci-dessus peut être mis en oeuvre pour, par exemple, déterminer l'état de tension de certains tendons, comme le tendon d'Achille, le tendon du triceps brachial ou encore le tendon rotulien ou ligament patellaire.

La mise en oeuvre du procédé selon l'invention sur des tissus humains ou animaux permet de mesurer directement les forces qui s'exercent sur différentes formations anatomiques, par exemple les efforts auxquels sont soumis les tendons, les ligaments et les muscles dans diverses circonstances, c'est-à-dire durant une locomotion normale ou une locomotion pathologique ou encore au cours de différents gestes (notamment gestes sportifs), que ce soit sur des tissus sains, des tissus lésés ou encore après appareillage des membres avec des prothèses.

La mise en oeuvre du procédé selon invention peut être réalisée au cours d'examens cliniques, d'évaluation de l'efficacité de certaines thérapies chimiques, mécaniques ou ré-éducatives.

Elle peut également être utile dans la conception de nouveaux matériels sportifs, ré-éducatifs ou médicaux, comme des chaussures de sport, des palmes de plongée, des fixations de ski, des appareils de musculation.

Le procédé selon l'invention peut aussi être appliqué à la caractérisation des lésions d'un tendon, d'un ligament ou d'un muscle ou encore à la détermination de l'étendue de telles lésions.

A titre illustratif, le procédé de mesure en continu de l'état de tension d'un tendon, d'un muscle ou d'un ligament défini ci-dessus peut être appliqué aux chevaux de course. En effet, les tendons sont des formations dont l'architecture est hautement spécialisée et adaptée à la résistance en traction. Toute lésion entraîne une perte de cette organisation et le processus de cicatrisation, particulièrement lent, est en général incomplet et ne permet pas au tendon de retrouver ses propriétés structurales et mécaniques d'origine. Les propriétés mécaniques d'un tendon lésé évoluent au cours de la cicatrisation. Pour une lésion récente, le tendon - malgré sa section augmentée - est fragile et se laisse déformer. Lorsque la lésion est ancienne, l'hypertrophie résiduelle, associée à une plus grande rigidité, rend comparativement plus fragiles les segments adjacents à la lésion initiale, au niveau desquels ont lieu en général les récidives. Dans la phase de cicatrisation, les chevaux sont classiquement appareillés avec des ferrures correctrices orthopédiques qui doivent être adaptées pour limiter les charges de tension du tendon cicatrisant du fait de leurs effets sur les configurations articulaires digitales. Afin de favoriser la vitesse et le niveau de cicatrisation, les principes suivants doivent en préférence être observés :
1) Au cours de la première phase, soit pendant les quatre à six premières semaines d'évolution de la lésion tendineuse, il convient de sélectionner une ferrure correctrice capable de soulager au maximum le tendon atteint en réduisant la tension subie par le tendon, pour éviter une aggravation et favoriser la cicatrisation ;
2) puis au cours de la phase suivante, s'étendant du deuxième au quatrième mois environ, il est préférable de chercher à solliciter légèrement le tendon pour (i) favoriser le réalignement longitudinal des fibres et (ii) éviter les rétractions cicatricielles. Il sera judicieux de sélectionner une ferrure capable d'induire un allongement progressif du tendon, alors que la cicatrisation progresse, de façon à améliorer de la déformabilité du tissu cicatriciel. L'objectif final est de réduire le risque de récidive ultérieure car des segments courts et épais prédisposent le tendon aux récidives à la jonction entre tissus sains et tissus lésés ;
3) enfin, lors de la reprise de l'activité sportive, en général au-delà d'une période de 4 à 6 mois après la lésion, il est nécessaire de sélectionner une ferrure capable d'épargner autant que possible le tendon, qui est le plus sujet aux récidives à ce stade.

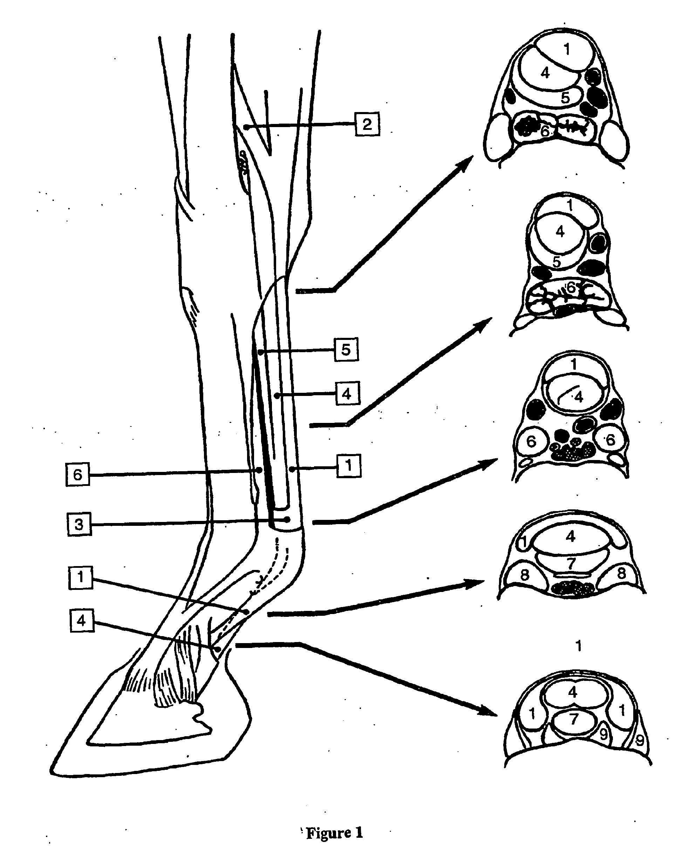

Le procédé de mesure en continu de l'état de tension d'un tendon, d'un muscle ou d'un ligament selon l'invention, lorsqu'il est appliqué aux équidés, est préférentiellement mis en oeuvre pour déterminer l'état de tension du tendon fléchisseur superficiel du doigt (TFSD) ou de son ligament accessoire (LA-TFSD), du tendon fléchisseur profond du doigt (TFPD) ou de son ligament accessoire (LA-TFPD), ou encore du muscle interosseux III, qui sont illustrés à la figure 1.

Ainsi, l'invention a également pour objet l'application du procédé de mesure en continu de l'état de tension d'un tendon tel que défini ci-dessus à la détermination de la tension d'un tendon d'équidé pendant la marche, le trot ou le galop.

Elle est également relative à un procédé pour sélectionner une ferrure correctrices destinée à modifier la tension d'un tendon chez un équidé, ledit procédé comprenant les étapes suivantes :
a) mettre en oeuvre le procédé de mesure en continu de l'état de tension d'un tendon, ci-dessus sur ledit tendon, après avoir appareillé l'équidé avec une ferrure correctrice ;
b) sélectionner la ferrure correctrice afin de modifier l'état de tension du tendon.

Par « modifier » l'état de tension du tendon, on entend augmenter ou réduire l'état de tension selon les objectifs spécifiques qui sont recherchés.

De préférence, l'étape a) du procédé de sélection ci-dessus est réalisée successivement avec une pluralité de ferrures correctrices, permettant ainsi de sélectionner la ferrure correctrice la plus appropriée compte-tenu des objectifs de soulagement de la charge de tension poursuivie.

L'invention est en outre illustrée, sans pour autant être limitée, par les figures et les exemples qui suivent.

### DESCRIPTION DES FIGURES

La **figure 1** représente schématiquement l'appareil musculo-tendineux de la main du cheval.
1) Tendon fléchisseur superficiel du doigt (TFSD).
2) Ligament accessoire du TFSD (LA-TFSD).
3) Manica flexoria.
4) Tendon fléchisseur profond du doigt (TFPD).
5) Ligament accessoire du TFPD (LA-TFPD).
6) Muscle interosseux III.
7) Ligament sésamoïdien droit (plan superficiel).
8) Ligaments sésamoïdiens obliques (plan moyen).
9) Ligaments palmaires de l'articulation interphalangienne proximale.

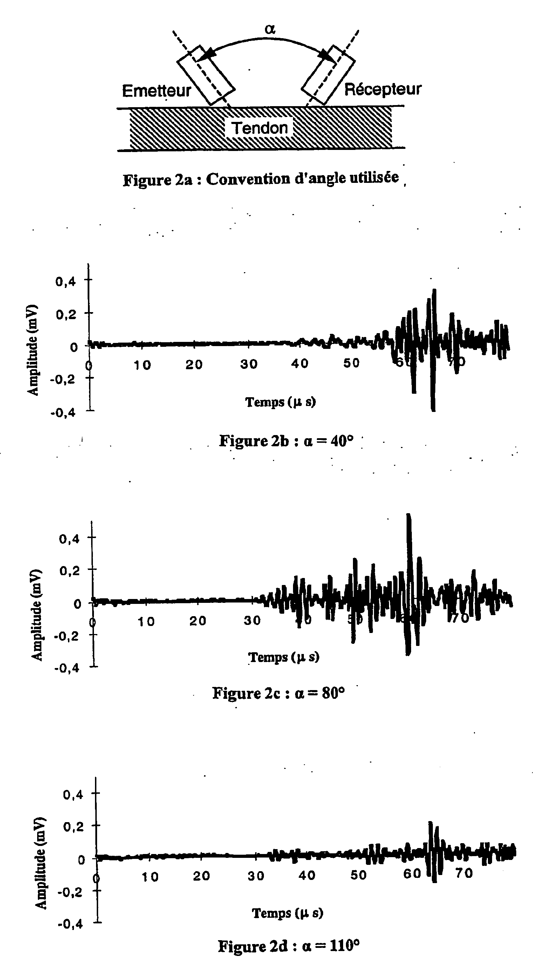

La **figure 2** illustre l'influence de l'angle entre l'émetteur et le récepteur sur le signal ultrasonore enregistré. La figure 2a présente la convention d'angle utilisée. L'angle inter-transducteurs α correspond à l'angle formé par les deux transducteurs placés symétriquement par rapport à la normale à la peau. Les figures 2b, 2c et 2d présentent les signaux ultrasonores recueillis pour un angle inter-transducteurs valant respectivement α = 40°, α = 80° et α = 110°. L'axe des abscisses représente le temps, exprimé en microseconde. L'axe des ordonnées représente l'amplitude du signal enregistré par le récepteur, exprimé en millivolts.

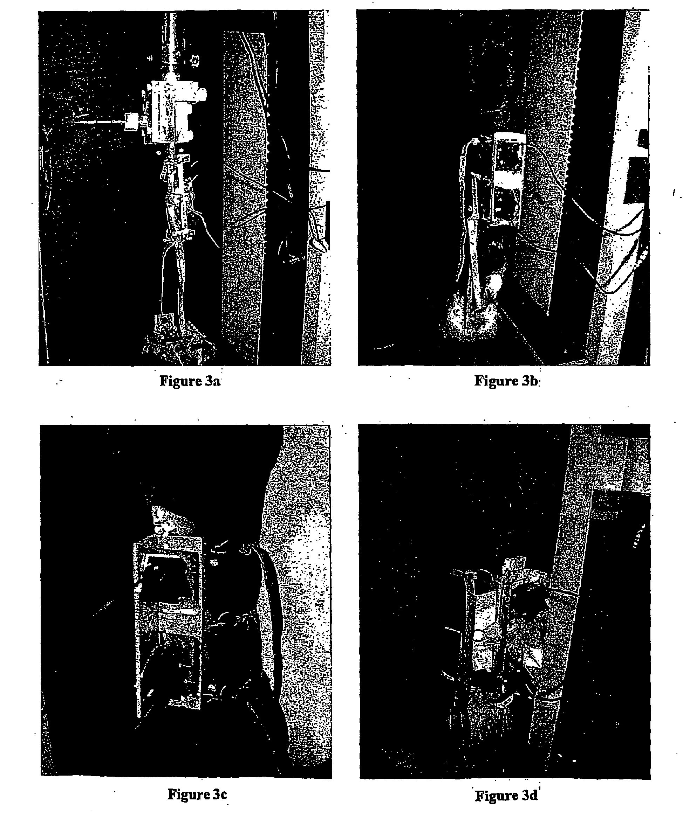

La **figure 3** illustre quatre des cinq conditions expérimentales présentées dans ce document. La figure 3A montre une vue du dispositif expérimental présenté, ci-dessous, dans l'exemple 1. Le tendon fléchisseur profond du doigt (TFPD) a été fixé proximalement dans une pince cryogénique et distalement par son insertion osseuse sur la phalange distale située au sein du sabot puis soumis à un test de traction entre 0 et 5000 N. Les figures 3B, 3C et 3D illustrent respectivement les expérimentations a) (peau intacte), c) (peau retirée) et d) (peau retirée et TFSD isolé acoustiquement des formations sous jacentes). Sur ces 4 illustrations on distingue les deux transducteurs (en noir) montés sur le système de contention (en gris clair) qui permet de les maintenir alignés selon l'axe principal du tendon et de leur imposer un angle prédéterminé par rapport à ce dernier.

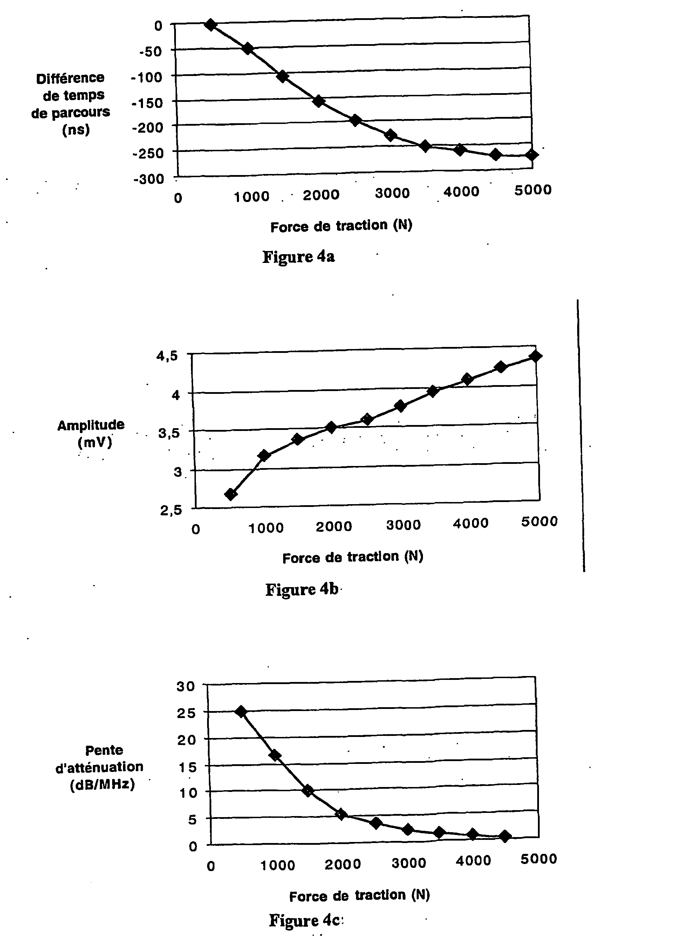

La **figure 4** illustre l'évolution du temps de parcours (figure 4A), de l'amplitude (figure 4B), et de l'atténuation (figure 4C) des ultrasons en fonction de la force de traction exercée sur un tendon FPD isolé. L'axe des abscisses représente la force de traction exercée sur le tendon FPD, exprimé en Newton (N). L'axe des ordonnées représente la variation de temps de parcours du signal ultrasonore exprimé en nanosecondes (ns) (figure 4A), l'amplitude du signal exprimé en millivolts (mV) (figure 4B) et son atténuation exprimée en décibel par mégaHertz (dB/MHz) (figure 4C).

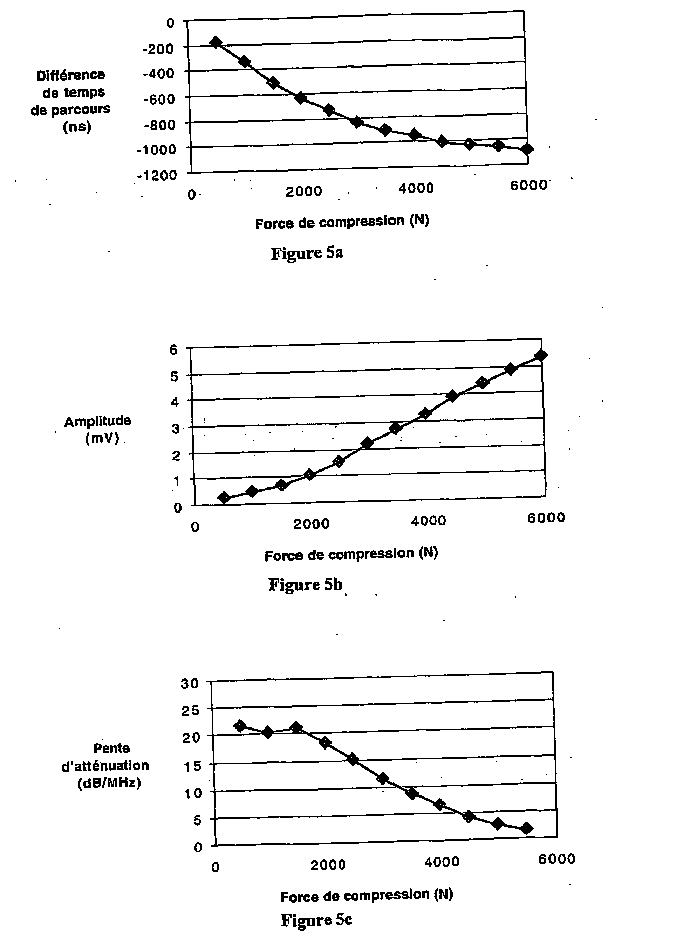

La **figure 5** illustre l'évolution du temps de parcours (figure 5A), de l'amplitude (figure 5B) et de l'atténuation (figure 5C) des ultrasons en fonction de la force de compression exercée sur un membre entier, les transducteurs étant disposés sur la peau en regard du tendon FSD, la peau étant tondue et intacte. L'axe des abscisses représente la force de compression exercée sur le membre, exprimée en Newton (N). L'axe des ordonnées représente la variation de temps de parcours du signal ultrasonore, exprimé en nanosecondes (ns) (figure 5A), l'amplitude du signal exprimé en millivolts (mV) (figure 5B) et son atténuation exprimée en décibel par mégaHertz (dB/MHz) (figure 5C).

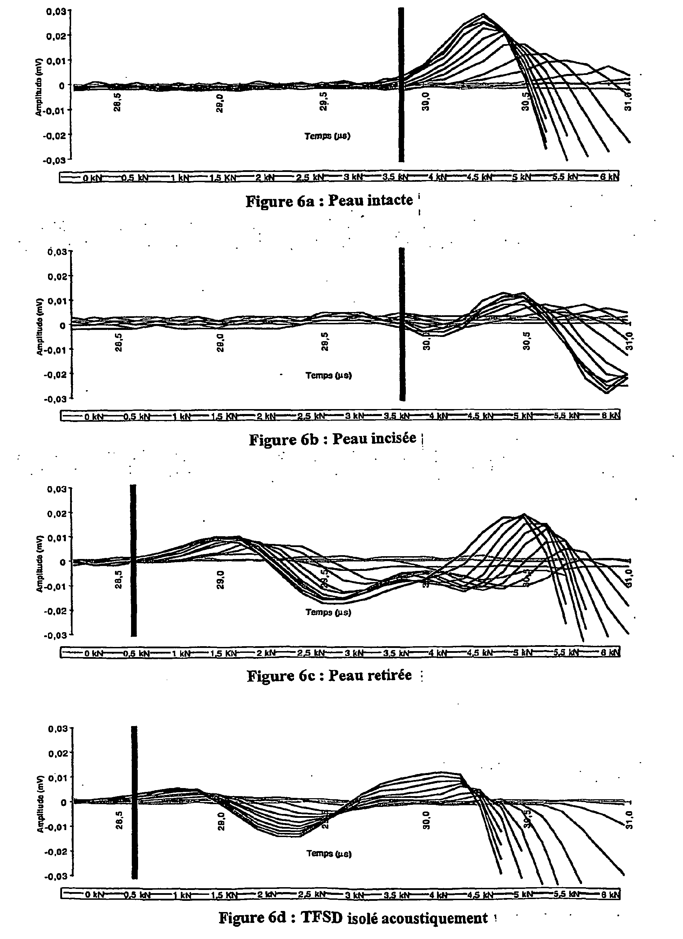

La **figure 6** illustre l'évolution du signal ultrasonore recueilli par le récepteur en fonction de la force de compression exercée sur un membre entier. Les figures 6A, 6B, 6C et 6D présentent quatre exemples de tracés obtenus respectivement dans les quatre conditions expérimentales testées c'est à dire peau intacte (figure 6A), peau incisée (figure 6B), peau retirée (figure 6C) et tendon fléchisseur superficiel du doigt isolé acoustiquement des formations anatomiques sous jacentes. Les différents tracés visibles sur ces figures correspondent aux signaux ultrasonores enregistrés pour chaque valeur d'effort exprimée en kiloNewton (kN). L'axe des abscisses représente le temps en microsecondes et l'axe des ordonnées l'amplitude du signal en millivolts. L'axe des ordonnées a été dilaté pour faciliter la détection visuelle du temps d'apparition des premières ondes ultrasonores (matérialisé par un trait vertical noir sur chaque figure).

La **figure 7** est le schéma d'un transducteur permettant la mise en oeuvre du procédé selon l'invention. La figure 7A est une vue du dessus du transducteur comprenant un émetteur et 5 récepteurs. La figure 7B représente une coupe longitudinale du transducteur et la figure 7C une vue de côté. (10) est l'émetteur d'ultrasons ; (11) à (15) représentent respectivement les récepteurs d'ultrasons R1 à R5.

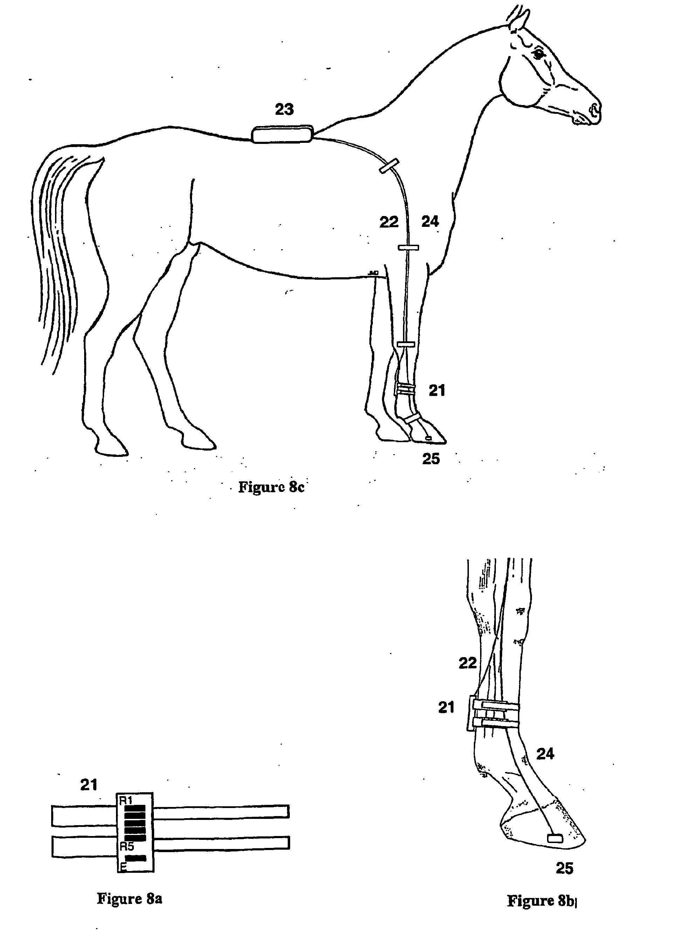

La **figure 8** est un schéma illustratif d'un dispositif complet permettant la mise eh oeuvre du procédé de mesure en continu selon invention. La figure 8A représente le transducteur (21) et son système de contention au membre. La figure 8B représente le détail d'un membre antérieur de cheval sur lequel à été fixé le transducteur (21) à l'aide du système de contention illustré figure 8A et un accéléromètre (25) permettant la détection du poser et du lever du membre au cours du mouvement. Le transducteur (21) et l'accéléromètre (25) sont reliés à un dispositif d'enregistrement de mesure par l'intermédiaire des câbles électriques ou optiques (22) et (24). La figure 8C est une vue d'ensemble du dispositif complet équipant un cheval. Le transducteur (21) et l'accéléromètre (25) sont reliés à un boîtier (23) fixé sur le dos de l'animal. Ce boîtier (23) contient l'électronique de commande du transducteur, l'électronique de traitement des signaux ultrasonores et le système de stockage de l'information.

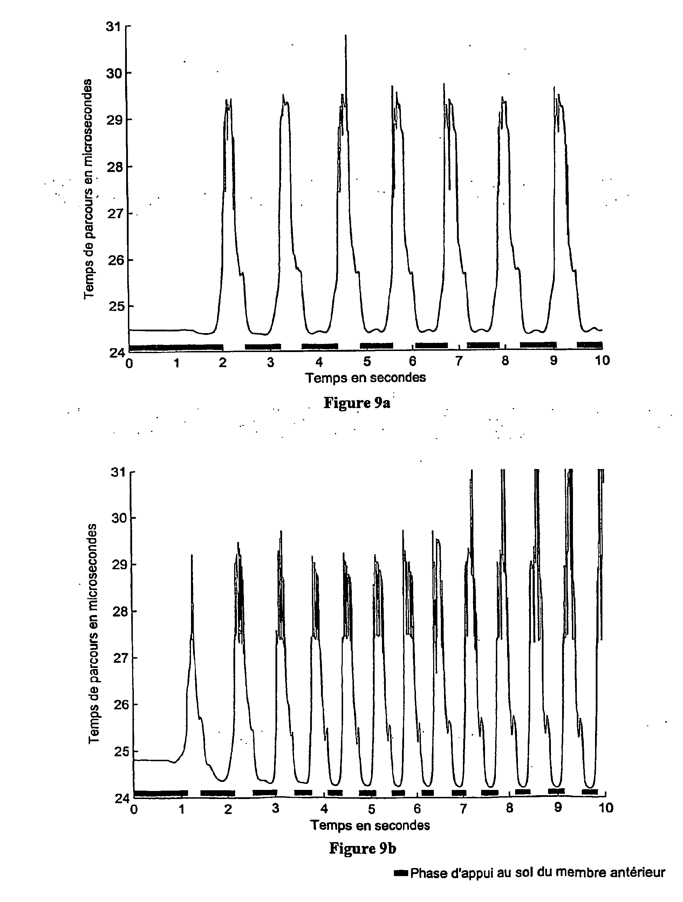

La **figure 9** illustre le temps de parcours des ultrasons entre le transducteur émetteur et le transducteur récepteur R5, après application du dispositif de la figure 8 en regard du tendon FSD d'un cheval.

Figure **9A** cheval au pas.

Figure **9B** **:** cheval au trot.

Au temps t = 0, le cheval est à l'arrêt. A partir du temps t = 2,4 secondes, le cheval est au pas. Les barres naines sur l'axe des abscisses représentent les moments d'appui au sol du membre antérieur.

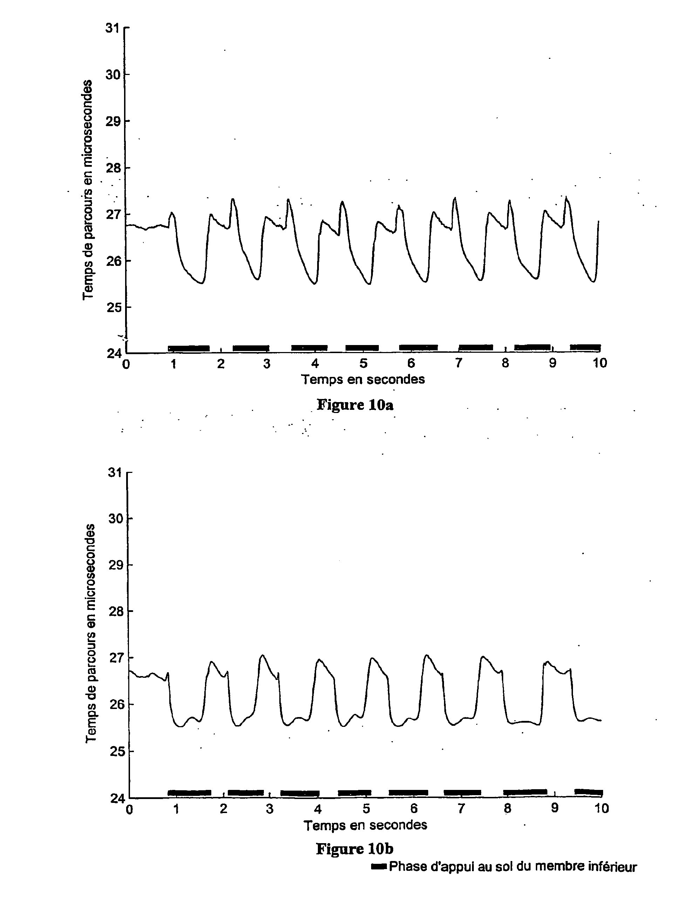

La figure **10** illustre le temps de parcours des ultrasons entre le transducteur émetteur et le transducteur récepteur R5, après application du dispositif de la figure 8 en regard du tendon d'Achille chez un homme.

Figure **10A** **:** homme au pas ; figure **10B** **:** homme montant un escalier.

Les barres noires sur l'axe des abscisses représentent les moments d'appui au sol du membre inférieur.

### EXEMPLES

### EXEMPLE 1 : Mesure de l'évolution du temps de parcours, de l'amplitude et de l'atténuation d'une onde ultrasonore sur un tendon isolé soumis à des charges de traction croissantes.

### a) MATÉRIELS ET MÉTHODES

### A.1 Le dispositif de mesure

Le dispositif de mesure se compose de deux transducteurs ultrasonores (un émetteur et un récepteur) reliés à un boîtier composé de cartes électroniques lui-même relié à un ordinateur numérique.
- L'émetteur et le récepteur sont de forme cylindrique, l'élément vibrant étant, dans les deux cas, un disque de 12mm de section, non focalisé, large bande (100 %) autour d'une fréquence centrale de 1MHz.
- Le boîtier se compose des cartes électroniques suivantes :
   - une carte d'émission générant une impulsion électrique large bande à 200V, envoyée sur le transducteur émetteur,
   - une carte d'acquisition et d'amplification à gain variable (24 à 66 dB),
   - une carte de conversion analogique/ numérique assurant d'une part, la numérisation du signal de réception amplifié, et d'autre part, l'interface avec l'ordinateur numérique pour le transit des commandes et des données.
- L'ordinateur numérique dispose d'un logiciel de gestion de l'ensemble de l'instrumentation. Ce logiciel assure :
   - l'interface avec l'utilisateur,
   - le lancement et la gestion des mesures,
   - le stockage des données obtenues.

Les deux transducteurs ont été fixés sur un système de contention au membre permettant de les maintenir alignés selon l'axe principal du tendon (figure 3A). Afin d'assurer un bon contact acoustique avec la peau, un gel aqueux a été utilisé comme couplant ultrasonore. Les ultrasons ont alors été émis puis recueillis après qu'ils se soient propagés dans le tendon.

Plus spécifiquement, les deux transducteurs fixés au système de contention ont été placés sur la face palmaire d'un tendon fléchisseur profond du doigt (TFPD) d'un cheval, après que celui-ci ait été isolé de son membre. Le TFPD est désigné par la référence (4) sur la figure 1.

Le TFPD a été soumis à un test en traction de 0 à 5000 N. Le tendon a été fixé proximalement dans une pince cryogénique et distalement par son insertion osseuse sur la phalange distale située au sein du sabot.

### b) RÉSULTATS

### B.1 Influence de l'angle formé par les transducteurs sur le signal ultrasonore

L'influence de l'angle formé par l'émetteur et le récepteur sur l'amplitude du signal ultrasonore lors de l'apparition de ce dernier a été étudié. Cette étude a été réalisée en faisant varier l'angle inter-transducteurs α (voir figure 2A) de 0° à 110° par incrément de 10°. Les résultats obtenus pour un angle α de 40°, 80° et 110° sont illustrés sur les figures 2B, 2C et 2D. Les résultats de cette étude ont montré que bien que le signal était détecté quelle que soit la valeur de l'angle, l'amplitude maximale du signal, en particulier au moment de son apparition, a été obtenue pour un angle inter- transducteurs de 80°.

En conséquence, la suite des expérimentations présentée dans les exemples a été réalisée avec un angle de 80° entre l'émetteur et le récepteur.

### B.2 Mesure des variation de temps de parcours, d'amplitude et d'atténuation des ultrasons en fonction de l'effort de traction appliqué au tendon

Comme cela est illustré à la figure 4, le temps de parcours (figure 4A) et l'atténuation (figure 4C) des ultrasons dans le tissu tendineux diminuent à mesure que la charge de traction exercée sur le tendon augmente. A l'inverse, l'amplitude (figure 4B) du signal ultrasonore augmente avec la charge de traction.

Ainsi, pour une traction de 1000 N, les valeurs de variation de temps de parcours (variation par rapport au temps de parcours observé à 500 N), d'amplitude et d'atténuation sont respectivement de -54 ns, 3,16 mV et 24,56 dB/MHz alors que pour une traction de 4000 N, ces variables valent respectivement -262 ns, 4,08 mV et 0,69 dB/MHz.

### EXEMPLE 2 : Mesure de l'évolution du temps dé parcours, de l'amplitude et de l'atténuation d'une onde ultrasonore sur le tendon fléchisseur superficiel du doigt d'un membre entier soumis à des charges de compression croissantes.

### A. MATÉRIELS ET MÉTHODES

### A.1 Le dispositif de mesure

Le dispositif de mesure est conforme à celui décrit pour l'exemple 1.

Pour un essai sur membre entier, le membre antérieur d'un cheval a été isolé au tiers distal de l'humérus puis a été soumis à une force de compression variant de 0 à 6000 N. La compression du membre a pour effet d'imposer un effort de traction aux 3 tendons du membre. Le dispositif de mesure a été appliqué sur la face palmaire de la région métacarpienne moyenne du membre, en regard du tendon fléchisseur superficiel du doigt (TFSD), référencé (1) dans la figure 1.

Quatre conditions ont été testées successivement :
a) Peau tondue intacte (figure 3B) ;
b) peau incisée entre les deux transducteurs ;
c) peau retirée (figure 3C) ; et
d) TFSD isolé acoustiquement des structures sous jacentes (autres tendons et os métacarpiens) au moyen d'une feuille de papier insérée entre le TFSD et le TFPD (figure 3D).

### B. RÉSULTATS

Dans les essais de l'exemple 2 sur le membre entier, les tendons fléchisseurs du doigt sont progressivement étirés au fur et à mesure de l'accroissement de la force de compression du membre.

Comme cela avait été observé à l'exemple 1, plus la charge de compression exercée sur le membre entier est élevée, c'est-à-dire plus l'effort de traction imposé aux tendons est grand, et plus le temps de parcours (figure 5A) et l'atténuation (figure 5C) des ultrasons sont faibles et, à l'inverse, plus l'amplitude (figure 5B) du signal ultrasonore est élevée. Ces observations sont valables quelle que soit la condition expérimentale (peau intacte, incisée, retirée ou TFSD isolé acoustiquement).

La comparaison des temps de parcours moyens mesurés dans ces 4 conditions expérimentales (traits verticaux de la figure 6) montre qu'en présence de la peau, intacte ou incisée, le temps mis par les ultrasons pour parvenir au récepteur est plus long de 1,25 microseconde que lorsque la peau est retirée. Considérant que la vitesse de propagation des ultrasons dans la peau est de 1500 m/s, ce délai correspond à une épaisseur de peau d'environ 1 mm, ce qui est tout à fait cohérent avec les données anatomiques.

Par ailleurs aucune différence significative de ce temps de parcours moyen n'a été observée entre la condition c) (peau retirée) et la condition d) (peau retirée et TFSD isolé acoustiquement des formations sous jacentes).

On peut donc conclure de ces deux observations que les premiers ultrasons reçus par le récepteur ont bien circulé à travers le tendon le plus superficiel (TFSD) et non à travers la peau ou d'autres formations anatomiques.

### EXEMPLE 3 : Dispositif de mesure utilisable dans la mise en oeuvre du procédé selon invention.

Comme cela est illustré sur la figure 7B, l'émetteur (10) d'une part et les récepteurs (11 à 15) d'autre part, forment un angle choisi de manière à optimiser l'amplitude du signal ultrasonore enregistré. Le transducteur est relié à un boîtier électronique de commande, de traitement et de stockage du signal.

La figure **8** illustre un mode de réalisation représentatif de l'appareillage d'un cheval avec un dispositif complet de mesure de l'état de tension d'un tendon, d'un ligament ou d'un muscle pour la mise en oeuvre du procédé selon invention.

La figure **8A** représente le dispositif de contention du transducteur (21) permettant de maintenir, à l'aide de sangles, le transducteur en regard du tendon, du ligament ou du muscle investigué.

La figure **8B** illustre le membre antérieur d'un cheval équipé avec le dispositif (21) duquel part un câble électrique (22) permettant la transmission des commandes d'émission d'ultrasons et de réception des signaux ultrasonores recueillis par les récepteurs.

Dans le mode de réalisation de la figure 8B, le système comporte également un accéléromètre (25) duquel part un câble (24) de transmission du signal accéléromètrique.

La figure **8C** illustre un schéma d'ensemble d'un cheval équipé du dispositif de mesure complet, qui comprend également un boîtier (23) électronique de commande et de traitement du signal et qui comporte également des moyens de stockage de l'information issue des transducteurs (21) et (25).

### EXEMPLE 4 : Temps de parcours d'une onde ultrasonore, mesuré en regard du tendon Fléchisseur Superficiel du Doigt d'un cheval se déplaçant au pas et au trot.

### a) MATÉRIELS ET MÉTHODES :

### A.1 Dispositif de mesure

Le cheval a été équipé du dispositif de mesure décrit dans l'exemple 3. Comme illustré sur les figures 8B et 8C, l'électronique a été fixée sur le dos de 1 l'animal et l'accéléromètre sur son sabot droit. Le capteur ultrasonore a été disposé en regard du tendon FSD, contre la peau de la région métacarpienne moyenne du membre antérieur droit.

### A.2 Enregistrements

Deux enregistrements de 10 secondes, l'un au pas, l'autre au trot, ont été réalisés. Dans les 2 cas, l'enregistrement a été déclenché, le cheval étant tenu en main à l'arrêt. Après environ une seconde d'enregistrement, l'animal a été conduit, en ligne droite et sur un terrain dur, d'une part au pas et, d'autre part, progressivement au trot.

### A.3 Traitement des enregistrements

Pour calculer le temps de parcours de l'onde ultrasonore, les signaux enregistrés ont été traités selon la procédure décrite dans la présente description. Pour déterminer les périodes correspondant à la phase d'appui du membre au sol et à celle de soutien, les pics d'accélération provoqués par le poser et le lever du sabot ont été identifiés sur le signal accélérométrique.

### b) RÉSULTATS

Les figures 9A et - 9B présentent les temps de parcours de l'onde ultrasonore, obtenus sur le récepteur R5, respectivement au pas et au trot. A titre d'exemple, on peut citer comme résultats les temps de parcours minimaux observés au pas et au trot lors des 5 dernières foulées (c'est-à-dire une fois la vitesse du cheval stabilisée). Ils ont été, respectivement au pas et au trot, de 24.37 ± 0,02 µs et de 24,20 ± 0,02 µs.

Ces résultats montrent clairement que la valeur du temps de parcours de l'onde ultrasonore, entre un émetteur d'ultrasons et un récepteur d'ultrasons localisé à une distance fixe prédéterminée dudit émetteur, est inversement proportionnel à la charge de tension du tendon du cheval.

### EXEMPLE 5 : Temps de parcours d'une onde ultrasonore, mesuré en regard du tendon d'Achille d'un homme se déplacant en marchant (pas) et en montant un escalier.

### a) MATÉRIELS ET MÉTHODES :

### A.1 Dispositif de mesure

Le dispositif de mesure est identique à celui décrit dans les exemples 3 et 4. Le sujet a été équipé d'un sac à dos dans lequel a été placée l'électronique. L'accéléromètre a été fixé sur le talon droit du sujet et le capteur ultrasonore a été disposé, émetteur vers le bas, contre la peau en regard de son tendon d'Achille droit.

### A.2 Enregistrements

Deux enregistrements de 10 secondes, l'un au pas en ligne droite, l'autre au cours de la montée de plusieurs marches d'escalier, ont été réalisés. Contrairement à l'exemple précédent, l'enregistrement a été déclenché alors que le membre inférieur droit du sujet était en phase de soutien (ou phase pendulaire).

### A.3 Traitement des enregistrements

Pour calculer le temps de parcours de l'onde ultrasonore, les signaux enregistrés ont été traités selon la procédure décrite dans la présente description. Pour déterminer les périodes correspondant à la phase d'appui du membre au sol et à celle de soutien, les pics d'accélération provoqués par le poser et le lever du membre ont été identifiés sur le signal accélérométrique.

### b) RÉSULTATS :

Les figures 10A et 10B présentent les temps de parcours de l'onde ultrasonore obtenus sur le récepteur R5, respectivement lors du pas et de la montée de l'escalier. A titre d'exemple de résultats, le temps de parcours moyen observé sur 7 foulées de pas a été de 26,38 ± 0,54 µs contre 26,13 ± 0,56 µs pour 7 montées de marches successives.

Ces résultats montrent clairement que la valeur du temps de parcours de l'onde ultrasonore, entre un émetteur d'ultrasons et un récepteur d'ultrasons localisé à une distance fixe prédéterminée dudit émetteur, est inversement proportionnel à la charge de tension du tendon humain.

## Revendications

1. Procédé pour déterminer l'état de tension d'un matériau biologique choisi parmi un tendon, un ligament et un muscle à un instant donné, comprenant une étape au cours de laquelle on calcule la valeur d'au moins un paramètre extrait du signal ultrasonore reçu après propagation d'une onde ultrasonore émise dans ledit matériau entre une source d'ultrasons et au moins un récepteur d'ultrasons situé à une distance fixe prédéterminée de ladite source, la valeur du ou des parametre(s) variant avec l'état de tension du matériau, ladite variation du ou des paramètre(s) étant proportionnelle(s) ou inversement proportionnelle(s) à la variation de l'état de tension dudit matériau.

2. Procédé selon la revendication 1, **caractérisé en ce que** le ou les paramètre(s) calculé (s) à partir du signal ultrasonore est (sont) choisi (s) parmi les paramètres suivants :
(i) le temps de parcours de l'onde ultrasonore sur une distance prédéterminée dans ledit matériau;
(ii) l'amplitude de l'onde ultrasonore au point de déception du signal ultrasonore ;
(iii) l'atténuation de l'onde ultrasonore au point de réception du signal ultrasonore;
(iv) la fréquence moyenne de l'onde ultrasonore au point de réception du signal ultrasonore ;
(v) la fréquence maximale de l'onde ultrasonore au point de réception du signal sonore.

3. Procédé selon l'une des revendications 1 ou 2, **caractérisé en ce que** l'on calcule les valeurs pour au moins l'une des combinaisons de paramètres suivantes :
(i) le temps de parcours et l'amplitude de l'onde ultrasonore;
(ii) le temps dé parcours et l'atténuation de l'onde ultrasonore ;
(iii) l'amplitude et l'atténuation de l'onde ultrasonore ;
(iv) le temps de parcours, l'amplitude et l'atténuation de l'onde ultrasonore.

4. Procédé selon l'une des revendications 1 à 3, **caractérisé en ce qu'**il comprend les étapes suivantes :
a) appliquer une source d'ultrasons au contact du matériau à tester et enregistrer les signaux provenant de cette source d'ultrasons, après propagation dans ledit matériau à l'aide d'au moins un récepteur situé à une distance fixe prédéterminée de ladite source, la ligne reliant la source et le ou les récepteurs étant parallèle à l'axe longitudinal du matériau à tester.
b) déterminer l'état de tension du matériau en calculant la valeur du ou des paramètre(s) extrait(s) du signal ultrasonore à un instant donné.

5. Procédé selon la revendication 4, **caractérisé en ce que** la source d'ultrasons et le ou les récepteurs d'ultrasons sont placés au contact du matériau à tester et alignés selon l'axe principal dudit matériau.

6. Procédé selon l'une des revendications 1 à 3, ledit procédé comprenant les étapes suivantes :
a) appliquer une source d'ultrasons au contact de la peau en regard d'un tendon, d'un ligament ou d'un muscle et recevoir les signaux provenant de cette source d'ultrasons après propagation dans ledit matériau à l'aide d'au moins un récepteur situé à une distance fixe, prédéterminée de ladite source, la source et le ou les récepteurs étant alignés selon l'axe principal du tendon, du ligament ou du muscle investigué,
b) déterminer l'état de tension du tendon, du ligament ou du muscle en calculant la valeur du ou des paramètre (s) extrait(s) du signal ultrasonore à un instant donné.

7. Procédé selon la revendication 6, **caractérisé en ce que** pour un tendon, un ligament ou un muscle, la ligne reliant la source et le ou les récepteurs est parallèle à l'axe selon lequel sont alignées respectivement les fibres tendineuses, ligamentaires ou musculaires.

8. Procédé selon l'une des revendications 5 à 7, **caractérisé en ce que** les signaux provenant de la source d'ultrasons sont enregistrés par une pluralité de récepteurs située à une distance prédéterminée les uns par rapport aux autres et par rapport à la source d'ultrasons.

9. Procédé selon l'une des revendications 5 à 8, **caractérisé en ce que** la source d'ultrasons, d'une part, et le ou la pluralité de récepteurs, d'autre part, font entre eux un angle compris entre 0° et 180°, de préférence un angle entre 60° et 100°, et de manière tout à fait préférée un angle d'environ 80°.

10. Procédé selon l'une des revendications 1 à 9, **caractérisé en ce que** la fréquence des ultrasons émis par la source est comprise entre 15 KHz et 10 GHz, avantageusement entre 20 KHz et 100 MHz, de préférence entre 20 KHz et 50 MHz.

11. Procédé selon l'une des revendications 5 à 10, **caractérisé en ce que** le ou les paramètre(s) extrait(s) du signal ultrasonore reçu est (sont) calculé(s) entre au moins deux récepteurs.

12. Procédé de mesure en continu de l'état de tension d'un matériau, **caractérisé en ce qu'**on met en oeuvre le procédé selon l'une des revendications 1 à 10 pour une pluralité d'instants donnés, échelonnés dans le temps.

13. Procédé selon la revendication 12, **caractérisé en ce que** le procédé selon l'une des revendications 1 à 6 est mis en oeuvre à une fréquence d'échantillonnage supérieure ou égale à 2,5 fois celle de la fréquence à laquelle s'effectue l'évolution de l'état de tension.

14. Procédé selon l'une des revendications 1 à 13 à **caractérisée en ce que** la détermination de la tension d'un tendon, d'un ligaments ou d'un muscle est réalisée chez l'homme ou l'animal en mouvement, durant une locomotion normale ou pathologique, pendant un geste sportif.

15. Procédé selon la revendication 14 **caractérisée en ce que** le procédé est réalisé sur des tissus lésés ou après appareillage d'un membre avec une prothèse.

16. Procédé pour sélectionner une ferrure correctrice destinée à modifier la tension d'un tendon chez un équidé comprenant les étapes suivantes :
a) mettre en oeuvre la procédé selon l'une des revendications 12 ou 13 ledit tendon, après avoir appareillé l'équidé avec une ferrure correctrice, ou successivement avec une pluralité de ferrures correctrices ;
b) sélectionner la ferrure correctrice pour modifier l'état de tension du tendon.

## Claims

1. A method to determine the tension condition of a biological material selected from a tendon, a ligament and a muscle at a given time, comprising one step during which one calculates the value of at least one parameter extracted from the ultrasonic signal received after propagation of an ultrasonic wave transmitted in said material between an ultrasound source and at least one ultrasound receiver situated at a fixed predetermined distance from said source, the value of the parameter(s) varying with the tension condition of the material, said variation of the parameter(s) being proportional or reversely proportional to the variation in the tension condition of said material.

2. A method according to claim 1, **characterised in that** the parameter(s) calculated on the basis of the ultrasonic signal is (are) selected among the following parameters: (i) the travel time of the ultrasonic wave over a predetermined distance in said material; (ii) the amplitude of the ultrasonic wave at the reception point of the ultrasonic signal; (iii) the attenuation of the ultrasonic wave at the reception point of the ultrasonic signal; (iv) the frequency of the ultrasonic wave at the reception point of the ultrasonic signal; (v) the maximum frequency of the ultrasonic wave at the reception point of the sound signal.

3. A method according to claim 1, **characterised in that** one calculates the values for at least one of the following parameter combinations: (i) the travel time and the amplitude of the ultrasonic wave; (ii) the travel time and the attenuation of the ultrasonic wave; (iii) the amplitude and the attenuation of the ultrasonic wave; (iv) the travel time, the amplitude and the attenuation of the ultrasonic wave.

4. A method according to one of claims 1 to 3, **characterised in that** it comprises the following steps: a) application of an ultrasound source in contact with the material to be tested and record the signals from this ultrasound source after propagation in said material using at least one receiver situated at a fixed predetermined distance from said source, the line connecting the source and the receiver(s) being parallel to the longitudinal axis of the material to be tested. b) determination of the tension condition of the material by calculating the value of the parameter(s) extracted from the ultrasonic signal at a given time.

5. A method according to claim 4, **characterised in that** the ultrasound source and the ultrasound receivers are placed in contact with the material to be tested and aligned according to the main axis of said material.

6. A method according to one of claims 1 to 3, said method comprising the following steps: a) application of an ultrasound source in contact with the skin facing a tendon, a ligament or a muscle and reception of the signals from this ultrasound source after propagation in said material using at least one receiver situated at a fixed, predetermined distance from said source, the source and the receivers being aligned according to the main axis of the tendon, of the ligament or of the muscle under investigation, b) determination of the tension condition of the tendon, of the ligament or of the muscle by calculating the value of the parameter(s) extracted from the ultrasonic signal at a given time.

7. A method according to claim 6, **characterised in that** for a tendon, a ligament or a muscle, the line connecting the source and the receiver(s) is parallel to the axis along which are aligned respectively the tendinous, ligamentous or muscular fibres.

8. A method according to one of claims 5 to 7, **characterised in that** the signals from the ultrasound source are recorded by a plurality of receivers situated at a predetermined distance relative to one another and with respect to the ultrasound source.

9. A method according to claim one of claims 5 to 8, **characterised in that** the ultrasound source, on the one hand, and the plurality of receivers, on the other hand, form between themselves an angle ranging between 0.degree, and 180.degree., preferably an angle between 60.degree. and 100.degree., and quite preferably an angle of approximately 80.degree.

10. A method according to one of claims 1 to 9, **characterised in that** the frequency of ultrasounds transmitted by the source ranges between 15 KHz and 10 GHz, advantageously between 20 KHz and 100 MHz, preferably between 20 KHz and 50 MHz.

11. A method according to one of claims 5 to 10, **characterised in that** the parameter(s) extracted from the ultrasonic signal received is (are) calculated between at least two receivers.

12. A method for continuous measurement of the tension condition of a material, **characterised in that** one implements the method according to one of claims 1 to 10 for plurality of time-spread, given instants.

13. A method according to claim 12, wherein the method according to one of claims 1 to 6 is implemented at a sampling frequency greater than or equal to 2.5 times that of the frequency at which the tension condition evolves.

14. A method according to one of claims 1 to 13, **characterised in that** the determination of the tension of a tendon, of a ligament or of a muscle is realised in a man or an animal in motion, during normal or pathological locomotion, during a action.

15. A method according to claim 14, **characterised in that** the method is realised on damaged tissues or after fitting a limb with a prosthesis.

16. A method for selection of a corrective iron fitting intended to modify the tension of a tendon in equines comprising the following steps: a) implementing the method according to one of claims 1 or 13 on said tendon, after fitting the equine with a corrective iron fitting, or successively with a plurality of corrective iron fittings; b) selecting the corrective iron fitting in order to modify the tension condition of the tendon.

## Patentansprüche

1. Verfahren zur Bestimmung des Spannungszustands eines biologischen Materials, das aus einer Sehne, einem Band und einem Muskel ausgewählt ist, zu einem gegebenen Zeitpunkt, umfassend einen Schritt, bei dem der Wert wenigstens eines Parameters berechnet wird, den man aus dem Ultraschallsignal erhält, das nach Ausbreitung einer in das Material ausgesandten Ultraschallwelle zwischen einer Ultraschallquelle und wenigstens eine Ultraschallempfänger, der sich in einem festen vorbestimmten Abstand von der Quelle befindet, empfangen wird, wobei der Wert des oder der Parameter mit dem Spannungszustand des Materials variieren, wobei die Variation des oder der Parameter proportional oder umgekehrt proportional zur Variation des Spannungszustand des Materials ist.

2. Verfahren gemäß Anspruch 1, **dadurch gekennzeichnet, dass** der oder die aus dem Ultraschallsignal berechneten Parameter aus den folgenden Parametern ausgewählt ist bzw. sind:
(i) der Laufzeit der Ultraschallwelle über eine vorbestimmte Strecke in dem Material;
(ii) der Amplitude der Ultraschallwelle am Punkt des Empfangs des Ultraschallsignals;
(iii) der Dämpfung der Ultraschallwelle am Punkt des Empfangs des Ultraschallsignals;
(iv) der mittleren Frequenz der Ultraschallwelle am Punkt des Empfangs des Ultraschallsignals;
(v) der maximalen Frequenz der Ultraschallwelle am Punkt des Empfangs des Schallsignals.

3. Verfahren gemäß einem der Ansprüche 1 oder 2, **dadurch gekennzeichnet, dass** die Werte für wenigstens eine der folgenden Parameterkombinationen berechnet erden:
(i) Laufzeit und Amplitude der Ultraschallwelle;
(ii) Laufzeit und Dämpfung der Ultraschallwelle;
(iii) Amplitude und Dämpfung der Ultraschallwelle;
(iv) Laufzeit, Amplitude und Dämpfung der Ultraschallwelle.

4. Verfahren gemäß einem der Ansprüche 1 bis 3, **dadurch gekennzeichnet, dass** es die folgenden Schritte umfasst:
a) Bringen einer Ultraschallquelle in Kontakt mit dem zu testenden Material und Aufzeichnen der von dieser Ultraschallquelle stammenden Signale nach Ausbreitung in dem Material mit Hilfe wenigstens eines Empfängers, der sich in einem festen vorbestimmten Abstand von der Quelle befindet, wobei die Gerade zwischen der Quelle und dem oder en Rezeptoren parallel zur Längsachse des zu testenden Materials verläuft;
b) Bestimmen des Spannungszustands des Materials, indem man den Wert des oder der zu einem gegebenen Zeitpunkt von dem Ultraschallsignal erhaltenen Parameter berechnet.

5. Verfahren gemäß Anspruch 4, **dadurch gekennzeichnet, dass** die Ultraschallquelle und der oder die Ultraschallempfänger mit dem zu testenden Material in Kontakt gebracht und nach der Hauptachse des Materials ausgerichtet werden.

6. Verfahren gemäß einem der Ansprüche 1 bis 3, wobei das Verfahren die folgenden Schritte umfasst:
a) Bringen einer Ultraschallquelle in Kontakt mit der Haut über einer Sehne, einem Band oder einem Muskel und Empfangen der Signale, die aus dieser Ultraschallquelle stammen, nach Ausbreitung in dem Material mit Hilfe wenigstens eines Empfängers, der sich in einem festen vorbestimmten Abstand von der Quelle befindet, wobei die Quelle und der oder die Empfänger nach der Hauptachse der zu untersuchenden Sehne, des zu untersuchenden Bandes oder Muskels ausgerichtet werden;
b) Bestimmen des Spannungszustands der Sehne, des Bandes oder des Muskels, indem man den Wert des oder der zu einem gegebenen Zeitpunkt von dem Ultraschallsignal erhaltenen Parameter berechnet.

7. Verfahren gemäß Anspruch 6, **dadurch gekennzeichnet, dass** bei einer Sehne, einem Band oder einem Muskel die Gerade zwischen der Quelle und dem oder den Rezeptoren parallel zu der Achse verläuft, nach der die Sehnen-, Band- oder Muskelfasern ausgerichtet sind.

8. Verfahren gemäß einem der Ansprüche 5 bis 7, **dadurch gekennzeichnet, dass** die von der Ultraschallquelle stammenden Signale von einer Vielzahl von Empfängern aufgezeichnet werden, die sich in einem vorbestimmten Abstand voneinander und von der Ultraschallquelle befinden.

9. Verfahren gemäß einem der Ansprüche 5 bis 8, **dadurch gekennzeichnet, dass** die Ultraschallquelle einerseits und der oder die Vielzahl von Empfängern andererseits zwischen sich einen Winkel von 0° bis 180°, vorzugsweise einen Winkel von 60° bis 100° und besonders bevorzugt einen Winkel von ungefähr 80° einschließen.

10. Verfahren gemäß einem der Ansprüche 1 bis 9, **dadurch gekennzeichnet, dass** die Frequenz der von der Quelle ausgesandten Ultraschallwellen zwischen 15 kHz und 10 GHz, vorteilhafterweise zwischen 20 kHz und 100 MHz, vorzugsweise zwischen 20 kHz und 50 MHz, liegt.

11. Verfahren gemäß einem der Ansprüche 5 bis 10, **dadurch gekennzeichnet, dass** der oder die Parameter, die von dem empfangenen Ultraschallsignal erhalten werden, zwischen wenigstens zwei Empfängern berechnet wird bzw. werden.

12. Verfahren zur kontinuierlichen Messung des Spannungszustands eines Materials, **dadurch gekennzeichnet, dass** das Verfahren gemäß einem der Ansprüche 1 bis 10 zu einer Vielzahl von gegebenen, zeitlich gestaffelten Zeitpunkten durchgeführt wird.

13. Verfahren gemäß Anspruch 12, **dadurch gekennzeichnet, dass** das Verfahren gemäß einem der Ansprüche 1 bis 6 mit einer Abtastfrequenz durchgeführt wird, die größer oder gleich der 2,5-fachen Frequenz, ist, mit der die Entwicklung des Spannungszustands erfolgt.

14. Verfahren gemäß einem der Ansprüche 1 bis 13, **dadurch gekennzeichnet, dass** die Bestimmung der Spannung einer Sehne, eines Bandes oder eines Muskels bei einem in Bewegung befindlichen Menschen oder Tier im Verlaufe einer normalen oder pathologischen Fortbewegung während einer sportlichen Betätigung durchgeführt wird.

15. Verfahren gemäß Anspruch 14, **dadurch gekennzeichnet, dass** das Verfahren an verletzten Geweben oder nach Ausstattung einer Gliedmaße mit einer Prothese durchgeführt wird.

16. Verfahren zum Auswählen eines Korrekturbeschlags, der die Spannung einer Sehne bei einem Vertreter der Equidae modifizieren soll, die folgenden Schritte umfassend:
a) Durchführen des Verfahrens gemäß einem der Ansprüche 12 oder 13 mit der Sehne, nachdem der Equidae mit einem Korrekturbeschlag oder nacheinander mit einer Vielzahl von Korrekturbeschlägen versehen wurde;
b) Auswählen des Korrekturbeschlags, um den Spannungszustand der Sehne zu modifizieren.
